# EUROPÄISCHE PATENTSCHRIFT

(11) **EP 2 887 859 B1**
(45) Veröffentlichungstag und Bekanntmachung des Hinweises auf die Patenterteilung: **19.07.2017**
(21) Anmeldenummer: 13752897.2
(22) Anmeldetag: 23.08.2013
(51) Int. Cl.: B23K 1/00, B23K 1/20, H05K 5/00, H05K 5/02, A61B 5/00, H04Q 9/14

(54) **MEDIZINISCHES IMPLANTAT SOWIE VERFAHREN ZU DESSEN HERSTELLUNG**
MEDICAL IMPLANT AND METHOD FOR THE PRODUCTION THEREOF
IMPLANT MÉDICAL ET PROCÉDÉ DE PRODUCTION

(30) Priorität: 24.08.2012 DE 102012107835
(43) Veröffentlichungstag der Anmeldung: 01.07.2015
(73) Patentinhaber: CorTec GmbH, 79110 Freiburg (DE); Albert-Ludwigs-Universität Freiburg, 79085 Freiburg (DE)
(72) Erfinder: RICKERT, Jörn, 79104 Freiburg (DE); KOHLER, Fabian, 79098 Freiburg (DE); SCHÜTTLER, Martin, 79312 Emmendingen (DE)
(74) Vertreter: 2s | ip Schramm Schneider Patentanwälte Rechtsanwälte
(86) Internationale Anmeldenummer: PCT/EP2013/067526
(87) Internationale Veröffentlichungsnummer: WO 2014/029863

(56) Entgegenhaltungen:
- WO-A1-2012/126003
- US-A1- 2007 167 867

## Beschreibung

### Gebiet der Erfindung

Die Erfindung betrifft ein medizinisches Implantat für einen menschlichen oder tierischen Organismus, ein Gehäuse für ein medizinisches Implantat, ein Verfahren zur Herstellung eines medizinischen Implantats sowie ein System, welches ein erfindungsgemäßes medizinisches Implantat und eine mit dem medizinischen Implantat koppelbare Sende-/Empfangseinheit umfasst.

### Stand der Technik und Hintergrund der Erfindung

Es ist bekannt, medizinische Implantate, insbesondere aktive medizinische Implantate für einen menschlichen oder tierischen Organismus in einem Gehäuse anzuordnen, welches dann in den menschlichen oder tierischen Körper eingepflanzt wird. Die Implantatgehäuse werden dabei vorwiegend aus Metallschalen gefertigt. Ferner ist es bekannt, Implantate so auszugestalten, dass eine Kommunikation zwischen dem Implantat und einer externen Sende-/Empfangseinheit ermöglicht wird. Die Kommunikation wird hierbei über Funk oder Induktion abgewickelt.

Bei der Kommunikation mittels Induktion ist allerdings die Sendebandbreite durch die Trägerfrequenz eingeschränkt. Wird die Trägerfrequenz für eine hohe Bandbreite, d.h. ein hohes Datenaufkommen, hoch gewählt, so wird sie durch die Materialien des Gehäuses und/oder durch das biologische Gewebe gedämpft. Der so entstehende Verlust muss ausgeglichen werden, indem die Sendeleistung entsprechend erhöht wird, was sich nachteilig auf die Lebensdauer des Implantats auswirkt, wenn das Implantat mit einer Batterie betrieben wird. Eine erhöhte Sendeleistung kann sich auch negativ auf das das Implantat umgebende biologische Gewebe auswirken, wenn etwa aufgrund der erhöhten Sendeleistung das das Implantat umgebende Gewebe direkt oder indirekt durch die inhärenten elektrischen Verluste der elektronischen Schaltkreise erwärmt wird.

Ferner ist es bei dem aus dem Stand der Technik bekannten Implantatgehäusen nachteilig, dass Feuchtigkeit, etwa Körperflüssigkeit, in das Gehäuse eindringen kann, die die Implantat-Elektronik innerhalb des Gehäuses beeinträchtigen kann. Es ist bekannt, Implantate bzw. Implantatgehäuse so auszulegen, dass ein Leck, durch das die Feuchtigkeit eindringen kann, entsprechend klein sein muss. Die zu erwartende Lebenszeit des Implantats wird durch Vermessung des Lecks während der Fertigung abgeschätzt. Dies ist allerdings insbesondere dann nachteilig, wenn das Implantat so auszulegen ist, dass es während der Lebenszeit des Patienten nicht ausgetauscht werden soll und gegebenenfalls viele Jahrzehnte zuverlässig funktionieren muss. Dringt dennoch zuviel Feuchtigkeit in das Implantatgehäuse ein, kann es dann zu Funktionsstörungen oder gar zu einem Ausfall des Implantats kommen, was sich nachteilig auf die Gesundheit des Patienten auswirken kann.

US 2007/167867 betrifft ein medizinisches Implantat mit den Merkmalen gemäß dem Oberbegriff des Anspruchs 1.

### Aufgabe der Erfindung

Aufgabe der vorliegenden Erfindung ist es daher, Lösungen für ein medizinisches Implantat bereitzustellen, welche die aus dem Stand der Technik bekannten Nachteile zumindest teilweise vermeiden und welche eine hohe Bandbreite der Kommunikation zwischen dem Implantat und einer externen Sende-/Empfangseinheit bei gleichzeitig langer Lebensdauer des Implantats ermöglichen.

### Erfindungsgemäße Lösung

Diese Aufgabe wird erfindungsgemäß gelöst durch ein medizinisches Implantat für einen menschlichen oder tierischen Organismus, ein Gehäuse für ein medizinisches Implantat, ein Verfahren zur Herstellung eines medizinischen Implantats sowie ein System, welches ein medizinisches Implantat und eine mit dem medizinischen Implantat koppelbare Sende-/Empfangseinheit umfasst, nach den unabhängigen Ansprüchen. Vorteilhafte Ausgestaltungen und Weiterbildungen der Erfindung sind in den jeweiligen abhängigen Ansprüchen angegeben.

Bereitgestellt wird demnach ein Gehäuse für ein medizinisches Implantat, wobei eine Gehäusewandung des Gehäuses zumindest teilweise lichtdurchlässig, insbesondere infrarotlichtdurchlässig, ausgestaltet ist. Vorteilhaft ist es, wenn die lichtdurchlässige bzw. infrarotlichtdurchlässige Gehäusewandung oder zumindest Teile hiervon durchlässig für Licht mit einer Wellenlänge von etwa zwischen 700 nm und 1000 nm, vorzugsweise zwischen 850 nm und 900 nm, ist. Licht dieser Wellenlängen wird vom Körpergewebe am wenigsten stark absorbiert und eignet sich daher besonders gut zur Datenübertragung.

Die Informationen werden in an sich bekannter Weise übertragen, indem die Infrarotlichtquelle in schnellen Folgen an- und ausgeschaltet wird, was von dem jeweiligen Infrarotempfänger registriert wird. Als Protokoll kann das IrDA-Protokoll vorgesehen sein.

Durch die licht- bzw. infrarotlichtdurchlässige Gehäusewandung wird eine Kommunikation zwischen dem Implantat und einer externen Sende-/Empfangseinrichtung in bidirektionaler Weise mittels Infrarotstrahlung ermöglicht. Dadurch ist eine energieeffizientere und störungsfreiere Übermittlung mit höheren Datenraten möglich. Weil eine Infrarotübertragungseinheit in das Gehäuse des medizinischen Implantats integriert bzw. im Gehäuse angeordnet werden höheren Datenraten möglich. Weil eine Infrarotübertragungseinheit in das Gehäuse des medizinischen Implantats integriert bzw. im Gehäuse angeordnet werden kann und die Datenübertragung durch den licht- bzw. infrarotlichtdurchlässigen Bereich der Gehäusewandung hindurch erfolgt, kann das Gehäuse wesentlich luft-bzw. feuchtigkeitsdichter ausgestaltet werden, weil auf Durchbrüche in der Gehäusewandung für das Durchführen von Kabeln für die Kommunikation mit der externen Sende-/Empfangseinrichtung verzichtet werden kann.

Das Gehäuse, insbesondere der lichtdurchlässig ausgestaltete Bereich des Gehäuses weist eine Keramik, insbesondere Aluminiumoxid oder Zirkoniumoxid in amorpher, kristalliner und/oder teilkristalliner Form, eine Hochtemperatur-Mehrlagenkeramik mit einem Aluminiumoxid-Anteil, oder ein Glas bzw. eine Glaskeramik auf.

Der lichtdurchlässig ausgestaltete Bereich des Gehäuses bzw. der Gehäusewandung kann durch eine Abdeckung gebildet werden, die eine Öffnung in der Gehäusewandung verschließt. Die Abdeckung kann in einer vorteilhaften Ausgestaltung der Erfindung eines der vorgenannten Materialien aufweisen.

Bereitgestellt wird des weiteren ein medizinisches Implantat für einen menschlichen oder tierischen Organismus, welches ein Gehäuse mit einer Gehäusewandung, welche zumindest einen lichtdurchlässig, insbesondere infrarotlichtdurchlässig ausgestalteten Bereich aufweist, und eine im Gehäuseinneren angeordnete Infrarot-Sendeeinrichtung und/oder Infrarot-Empfangseinrichtung umfasst.

Weil die Gehäusewandung licht- bzw. infrarotlichtdurchlässig ausgestaltet ist, kann mittels Infrarotstrahlung eine Kommunikation zwischen dem Implantat und einer externen Sende-/Empfangseinheit durch die Gehäusewandung hindurch abgewickelt werden. Die Bandbreite der Kommunikationsverbindung kann dadurch wesentlich erhöht werden. Ein weiterer Vorteil liegt darin, dass die Handhabung und die Fertigung eines medizinischen Implantats durch die Integration sämtlicher kationseinheit, was ein kompakteres Systemdesign erlaubt und die Zuverlässigkeit erhöht, weil etwa mechanische Schwachpunkte an dem Gehäuse entfallen. Ferner kann aufgrund der durch die Infrarottechnologie realisierbaren Datenraten das Einsatzspektrum des Implantats erheblich erweitert werden und ermöglicht die Verwendung komplexerer Elektroniksysteme für aufwändigere Anwendungen.

Es hat sich als vorteilhaft herausgestellt, wenn der lichtdurchlässig ausgestaltete Bereich eine Keramik, insbesondere Aluminiumoxid oder Zirkoniumoxid in amorpher, kristalliner und/oder teilkristalliner Form, eine Hochtemperatur-Mehrlagenkeramik mit einem Aluminiumoxid-Anteil oder ein Glas bzw. eine Glaskeramik aufweist.

Es hat sich herausgestellt, dass diese Materialien bei unterschiedlichen Dicken zwischen etwa 150 µm und 2 mm eine genügend große Transparenz für InfrarotStrahlen aufweisen.

In einer Ausgestaltung der Erfindung kann der lichtdurchlässig ausgestaltete Bereich durch eine Abdeckung gebildet werden, die eine Öffnung in der Gehäusewandung verschließt. Damit kann auf besonders einfache Weise ein transparenter Bereich des Gehäuses hergestellt werden, der unterschiedliche Materialien aufweist als das übrige Gehäuse.

Es hat sich als vorteilhaft herausgestellt, wenn im Gehäuseinneren zumindest ein Sensor, insbesondere ein Feuchtigkeitssensor und/oder ein Temperatursensor und/oder ein Drucksensor angeordnet sind, der operativ mit der Infrarot-Sendeeinrichtung und/oder Infrarot-Empfangseinrichtung gekoppelt ist.

Dadurch wird in vorteilhafter Weise die Möglichkeit bereitgestellt, für den Zuverlässigen Betrieb ausschlaggebende Parameter der Umgebung der elektronischen Schaltung innerhalb des Gehäuses, etwa Feuchtigkeit, Druck oder Temperatur des Gehäuses bzw. des Gehäuseinneren aufzunehmen und über die Infrarot-Schnittstelle nach außen zu kommunizieren.

Vorteilhaft ist es, wenn in dem Gehäuseinneren zumindest ein Magnet, insbesondere Permanentmagnet, angeordnet ist, der mit einem außerhalb des Gehäuses anordenbaren Magneten einer externen Sende-/Empfangseinheit zusammenwirken kann. Damit kann eine einfache Ausrichtung der externen Sende-/Empfangseinheit zum medizinischen Implantat erfolgen.

Vorteilhafterweise erfolgt die Energieversorgung des Implantats mittels magnetischer Induktion, wobei ein zur kabellosen Übertragung der Energie benötigtes magnetisches Wechselfeld außerhalb des Körpers bereitgestellt wird. Das magnetische Wechselfeld wird nicht zur Übertragung von Daten verwendet, sodass das magnetische Wechselfeld zum Zwecke eines Datentransfers nicht moduliert werden muss und damit der Einsatz von Induktionsspulen mit sehr hoher Güte auf Senderseite und Empfängerseite (Implantat) ermöglicht wird. Damit wird eine besonders effiziente Kopplung zum Sender bzw. zur senderseitigen Induktionsspule möglich.

In einer vorteilhaften Ausgestaltung des Implantats kann die Induktionsspule durch einen am Implantatgehäuse umwickelten elektrischen Leiter gebildet werden. Hierbei ist es vorteilhaft, wenn das Implantatgehäuse aus einer Keramik gefertigt ist. Durch die Spule wird das magnetische Wechselfeld im Implantat in eine Spannung umgeformt.

Durch die Kombination von induktiver Energieversorgung des Implantats mit einer (bidirektionalen) IR-Datenübertragung ist ein energiesparender Einsatz des Implantats möglich, weil das Implantat beispielsweise erst mit Heranführen eines magnetischen Wechselfeldes an das Implantat aktiviert wird. Zudem sind das Implantat und insbesondere die Datenübertragung erheblich unempfindlicher gegen elektromagnetische Störungen. Ferner kann im Implantat eine interne Energieversorgung, etwa ein Akkumulator vorgesehen sein, der bei Bedarf mit der induktiv übertragenen Energie geladen werden kann. Ein weiterer Vorteil ist die Trennung von (bidirektionaler) Datenübertragung und Energieversorgung.
Als weiter vorteilhaft hat sich herausgestellt, wenn in dem Gehäuseinneren ein Trocknungsmittel, beispielsweise Getter-Materialien, vorgesehen ist. Damit kann die Feuchtigkeit im Gehäuseinneren reduziert werden, was zum Schutz der in dem Gehäuseinneren angeordneten elektronischen Komponenten beiträgt. Das Trocknungsmittel kann an der Innenwandung des Gehäuses aufgebracht sein.

In einer Ausgestaltung der Erfindung kann der lichtdurchlässig ausgestaltete Bereich des Gehäuses eine geringere Stärke aufweisen, als der restliche Bereich des Gehäuses. Damit kann ein stabiles Gehäuse bereitgestellt werden, welches lediglich im Bereich, der für die Infrarot-Kommunikation lichtdurchlässig ausgestaltet ist, eine geringere Stärke aufweist.

Ferner kann es vorteilhaft sein, wenn an der Innenwandung des Gehäuses eine elektrische Schirmung, etwa eine Metallfolie, angeordnet ist, wobei die elektrische Schirmung derart ausgestaltet ist, dass sie an vorbestimmten Stellen licht- bzw. infrarotlichtdurchlässig ausgestaltet ist.

Ferner kann es vorteilhaft sein, das Gehäuse mit licht- bzw. infrarotlichtdurchlässigen Materialien zu beschichten. In einer Ausgestaltung der Erfindung kann eine Kunststoffummantelung zum mechanischen Schutz und zur Erhöhung der Biokompatibilität des Implantats vorgesehen sein, welche einen licht- bzw. infrarotlichtdurchlässig ausgestalteten Bereich aufweist. Mit der Kunststoffummantelung kann auch die umwickelte Induktionsspule ummantelt werden. Hierbei kann eine mehrlagige Kunststoffschicht vorgesehen sein. In einer weiteren Ausgestaltung der Erfindung kann die Außenseite des Gehäuses mit Materialien beschichtet werden, die biologische Vorgänge, etwa Wundheilung, begünstigen.

Des Weiteren wird durch die Erfindung ein Verfahren zur Herstellung eines medizinischen Implantats, insbesondere eines erfindungsgemäßen Implantats bereitgestellt, wobei
- auf einer Oberseite eines Basissubstrats eine im Wesentlichen erste rahmenförmige Metallschicht bzw. metallische Schicht aufgebracht wird,
- auf der Oberseite des Basissubstrats und innerhalb der ersten rahmenförmigen metallischen Schicht eine Infrarot-Sendeeinrichtung und/oder Infrarot-Empfangseinrichtung angeordnet wird,
- auf der Oberseite des Basissubstrats ein Deckelsubstrat angeordnet wird, welches den Bereich innerhalb der ersten rahmenförmigen metallischen Schicht abdeckt und welches zumindest einen lichtdurchlässig, insbesondere infrarotlichtdurchlässig ausgestalteten Bereich aufweist, wobei an dem Deckelsubstrat eine zweite, mit der ersten rahmenförmigen metallischen Schicht korrespondierende, rahmenförmige metallische Schicht vorgesehen wird, und
- die erste rahmenförmige metallische Schicht derart mit der zweiten rahmenförmigen metallischen Schicht unlösbar verbunden wird, dass das Basissubstrat, das Deckelsubstrat und die rahmenförmigen metallischen Schichten zusammen ein im Wesentlichen hermetisch geschlossenes Gehäuse des Implantats bilden.

Hermetisch geschlossen bedeutet, dass das Gehäuse weitestgehend bzw. vollständig luftdicht und/oder wasserdicht bzw. feuchtigkeitsdicht ist, sodass es während des vorgesehenen Betriebszeitraumes des Implantats, der mehrere Jahre oder Jahrzehnte betragen kann, nicht zu feuchtigkeitsbedingten Fehlfunktionen des Implantats kommen kann.

Weil die Infrarot-Sendeeinrichtung und/oder Infrarot-Empfangseinrichtung im Inneren des Gehäuses angeordnet wird, kann die Herstellung eines Implantats wesentlich vereinfacht werden, weil auf Durchbrüche in der Gehäusewandung zur Datenkommunikation verzichtet werden kann, sodass bei der Herstellung eines Implantats keine Maßnahmen getroffen werden müssen, um derartige Durchbrüche luft- bzw. wasserdicht zu verschließen.

Zwischen der ersten rahmenförmigen metallischen Schicht und der zweiten rahmenförmigen metallischen Schicht kann ein umlaufender Rahmen, vorzugsweise Metallrahmen, angeordnet werden, wobei der umlaufende Rahmen mit den beiden metallischen Schichten unlösbar verbunden wird.

Der lichtdurchlässig ausgestaltete Bereich kann durch eine Abdeckung gebildet werden, mit der eine Öffnung in dem Deckelsubstrat verschlossen wird.

In einer Ausgestaltung der Erfindung kann das Deckelsubstrat so ausgestaltet werden, dass der lichtdurchlässige Bereich eine geringere Stärke aufweist, als der restliche Bereich des Deckelsubstrats.

Vorteilhaft ist es, wenn für den lichtdurchlässigen Bereich Keramik, insbesondere Aluminiumoxid oder Zirkoniumoxid in amorpher, kristalliner und/oder teilkristalliner Form, eine Hochtemperatur-Mehrlagenkeramik mit einem Aluminiumoxid-Anteil oder ein Glas bzw. eine Glaskeramik verwendet wird.

Im Inneren des Gehäuses kann zumindest ein Sensor insbesondere ein Feuchtigkeitssensor und/oder ein Temperatursensor und/oder ein Drucksensor angeordnet werden, der operativ mit der Infrarot-Sendeeinrichtung und/oder Infrarot-Empfangseinrichtung gekoppelt wird.

Alternativ oder zusätzlich kann im Inneren des Gehäuses ein Magnet, insbesondere Permanentmagnet, der mit einem außerhalb des Gehäuses anordenbaren Magneten einer externen Sende-/Empfangseinheit zusammenwirken kann, angeordnet werden.

Alternativ oder zusätzlich kann das Gehäuse mit einer Induktionsspule umwickelt werden, die als induktive Empfangsspule für eine induktive Energieübertragung vorgesehen ist. Vorteilhafterweise wird hierfür eine Induktionsspule mit sehr hoher Güte vorgesehen. Vorteilhaft ist es, wenn die Induktionsspule nur für die Energieübertragung verendet wird, nicht aber für eine kabellose Datenübertragung.

Ferner kann zusätzlich oder alternativ ein Trocknungsmittel im Inneren des Gehäuses angeordnet werden.

Ferner wird durch die Erfindung ein System, umfassend ein medizinisches Implantat für einen menschlichen oder tierischen Organismus, insbesondere ein erfmdungsgemäßes Implantat, und eine Sende-/Empfangeinheit bereitgestellt, wobei die Sende-/Empfangseinheit mit einer Infrarot-Sendeeinrichtung und/oder Infrarot-Empfangseinrichtung des Implantats zusammenwirkt. Die Sende-/Empfangseinheit kann eine Infrarot-Sendeeinrichtung und/oder eine Infrarot-Empfangseinrichtung aufweisen. Die Infrarot-Sendeeinrichtung und/oder Infrarot-Empfangseinrichtung des Implantats und die Infrarot-Sendeeinrichtung und/oder Infrarot-Empfangseinrichtung der Sende-/Empfangseinheit können durch einen lichtdurchlässig, insbesondere infrarotlichtdurchlässig, ausgestalteten Bereich des Gehäuses des Implantats hindurch operativ koppelbar sein.

### Kurzbeschreibung der Figuren

Weitere Einzelheiten und Merkmale der Erfindung sowie konkrete Ausführungsbeispiele der Erfindung ergeben sich aus der nachfolgenden Beschreibung in Verbindung mit der Zeichnung. Es zeigt:
- Fig. 1: die Schritte eines Herstellungsverfahrens eines medizinischen Implantats gemäß einer ersten Ausführungsform;
- Fig. 2: die Schritte eines Herstellungsverfahrens eines erfindungsgemäßen Implantats gemäß einer zweiten Ausführungsform;
- Fig. 3: ein Diagramm, aus dem die relative Transmission von Infrarotstrahlungen in Abhängigkeit von der Wellenlänge der Infrarotstrahlen und der verwendeten Materialien hervorgeht;
- Fig. 4: einen schematischen Aufbau eines erfindungsgemäßen medizinischen Implantats;
- Fig. 5: fünf verschiedene Ausführungsvarianten eines erfindungsgemäßen medizinischen Implantats; und
- Fig. 6: vier weitere Ausführungsvarianten eines erfindungsgemäßen medizinischen Implantats.

### Detaillierte Beschreibung der Erfindung

Gemäß der vorliegenden Erfindung erfolgt die Kommunikation zwischen einem medizinischen Implantat und einer außerhalb des Körpers anordenbaren Sende/Empfangseinheit auf Basis von Licht bzw. Infrarotlicht, wobei die Kommunikation bidirektional abgewickelt werden kann. Um dies zu ermöglichen, ist zumindest ein Teil des Implantatgehäuses licht- bzw. infrarotlichtdurchlässig ausgestaltet.

Die Kommunikation von Implantaten mit der Außenwelt, etwa mit einer außerhalb des Körpers angeordneten Sende/Empfangseinheit ist notwendig, um einerseits die Funktionsfähigkeit und den Zustand des Implantats prüfen zu können. Hierfür werden keine hohen Bandbreiten bzw. Informationsübertragungsraten benötigt.

Andererseits ist die Kommunikation bzw. Übertragung physiologischer Daten für verschiedene diagnostische und therapeutische Anwendungen notwendig. Solche Daten sind etwa Blutdruck, Temperatur, Hormon- oder andere Botenstoffkonzentrationen oder die elektrische Aktivität von Nerven oder Muskeln. Die Datenübertragung auf Basis von Licht bzw. Infrarotlicht hat sich hierbei als besonders vorteilhaft herausgestellt, denn diese Daten können leistungsstark, d.h., mit einem hohem Informationsgehalt, energiesparend und sicher, d.h., mit geringen Fehler-/Verlustraten übertragen werden, sodass diese Daten auch für die klinische Nutzung verwendbar sind.

Umgekehrt ist auch die Kommunikation der Außenwelt, etwa einer außerhalb des Körpers angeordneten Sende/Empfangseinheit mit dem Implantat möglich. Damit kann beispielsweise das Implantat an- oder ausgeschaltet werden oder es können dem Implantat neue Funktionsmodi mitgeteilt werden. Hierfür sind keine hohen Bandbreiten bzw. Informationsübertragungsraten nötig.

Gemäß der Erfindung ist es auch möglich, eine andauernde bis hin zu einer kontinuierlichen Kommunikation abzuwickeln, um in therapeutischen Anwendungen flexibel und schnell reagieren zu können. Beispielsweise kann dadurch die Abgabe von Wirkstoffen schnell und zustandsabhängig gesteuert werden, Mikropumpen können bedarfsabhängig gesteuert werden, oder eine elektrische Stimulation von Nerven oder Muskeln kann schnell und bedarfsgerecht umgesetzt werden. Beispielsweise sind die bedarfsgerechte Neurostimulation bei Parkinson-Patienten oder die reaktive Stimulation bei Epilepsiepatienten, wenn ein drohender Anfall gemessen wird, möglich.

Im Gegensatz zu einer im Inneren des Implantats festgelegten Reaktionsweise bringt die Steuerung von außen zwei wesentliche Vorteile. Ein erster Vorteil liegt darin, dass ein Arzt oder der Patient selbst jederzeit die Aktivität des Implantats anpassen kann. Beispielsweise kann ein Patient die Wirkung des Implantats durch direkte Steuerung der äußeren Sendeeinheit umgehend anpassen. Ein zweiter Vorteil liegt darin, dass die Aktivität des Implantats mit Hilfe von Informationen von außen gesteuert werden kann. Beispielsweise kann die Aktivität einer Sehprothese durch die Lichtstärke (drinnen/draußen, Tag/Nacht) automatisch hoch- oder runterreguliert werden.

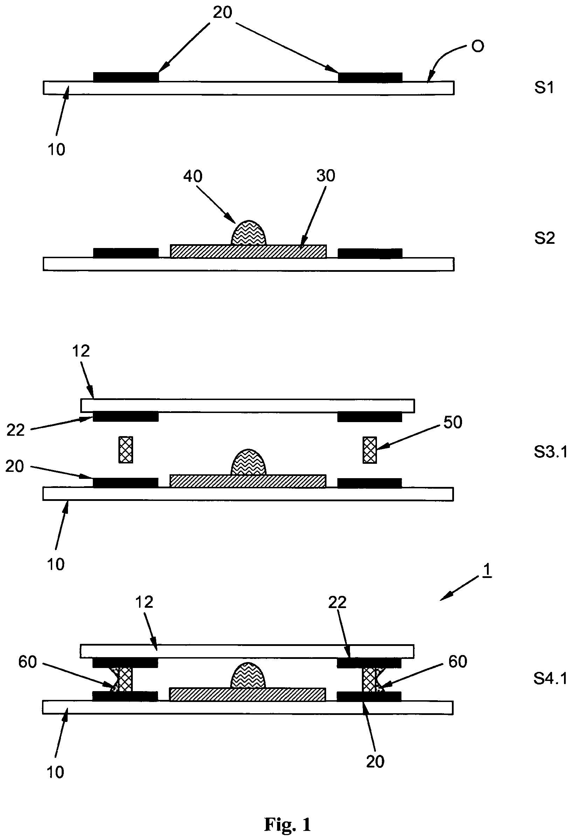

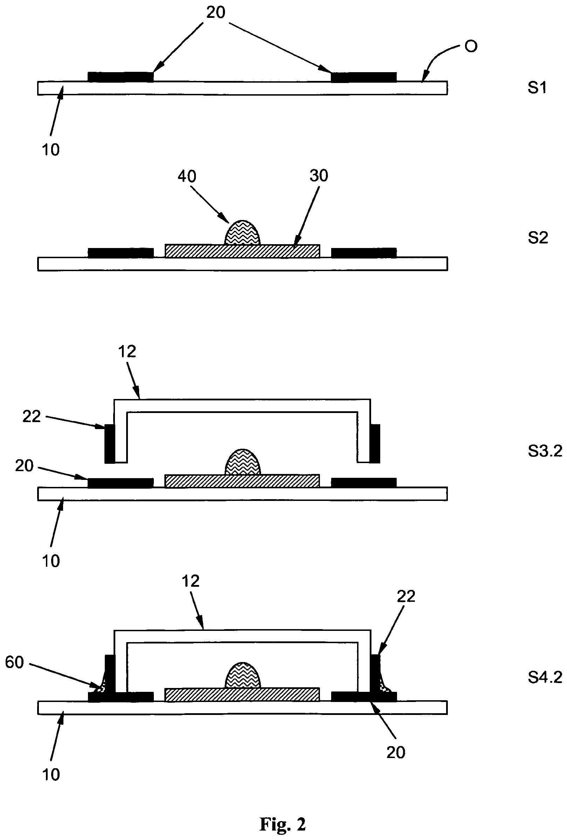

**Fig. 1** und **Fig. 2** zeigen die wesentlichen Verfahrensschritte zweier erfindungsgemäßer Verfahren zur Herstellung eines medizinischen Implantats. Die ersten beiden Verfahrensschritte S1, S2 sind bei dem Herstellungsverfahren gemäß einer ersten Variante (Fig. 1) identisch zu den beiden ersten Verfahrensschritten S1, S2 eines erfindungsgemäßen Herstellungsverfahrens gemäß einer zweiten Variante (Fig. 2).

In einem ersten Schritt S1 wird auf der Oberseite O eines Basissubstrats 10 eine metallische Schicht bzw. eine Metallisierungsschicht aufgebracht, welche im Wesentlichen eine erste rahmenförmige metallische Schicht 20 bildet. Die erste rahmenförmige metallische Schicht 20 dient als Lötbasis für weitere Elemente des Gehäuses des medizinischen Implantats.

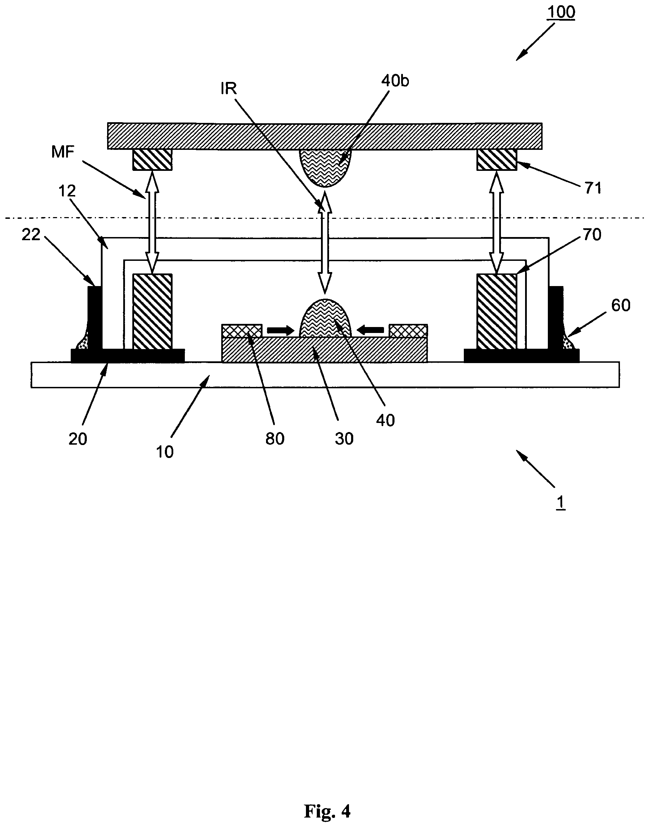

Im folgenden Herstellungsschritt S2 werden sämtliche Elektronikkomponenten auf der Oberseite O des Basissubstrats 10 und innerhalb der ersten rahmenförmigen metallischen Schicht 20 angeordnet und auf dem Basissubstrat 10 fixiert. Die Elektronikkomponenten umfassen hier eine Platine 30 und eine auf der Platine 30 angeordnete Infrarot-Sendeeinrichtung und/oder Infrarot-Empfangseinrichtung 40, wobei die Infrarot-Sende/Empfangseinrichtung hier eine Infrarot-Diode umfasst. Auf der Platine 30 können weitere elektronische Komponenten, etwa Sensoren, beispielsweise Temperatursensoren, Feuchtigkeitssensoren oder Drucksensoren, angeordnet werden, wie mit Bezug auf Fig. 4 näher erläutert wird.

In einem dritten Schritt S3.1 des Herstellungsverfahrens gemäß der in Fig. 1 gezeigten ersten Variante wird auf der Oberseite O des Basissubstrats 10 ein Deckelsubstrat 12 angeordnet. Das Deckelsubstrat 12 deckt den Bereich innerhalb der ersten rahmenförmigen metallischen Schicht 20 ab. An dem Deckelsubstrat 12 bzw. an der Unterseite des Deckelsubstrats 12 ist eine zweite rahmenförmige metallische Schicht 22 vorgesehen, welche mit der ersten rahmenförmigen metallischen Schicht 20 des Basissubstrats 10 korrespondiert. Zwischen der ersten rahmenförmigen metallischen Schicht 20 und der zweiten rahmenförmigen metallischen Schicht 22 wird zudem ein umlaufender Rahmen 50 angeordnet, der im Wesentlichen die Seitenwandung des Implantatgehäuses bildet. Der umlaufende Rahmen 50 kann aus einem Metall gefertigt sein.

In einem weiteren Schritt S4.1 der ersten Variante des Herstellungsverfahrens wird der umlaufende Rahmen 50 auf die erste umlaufende metallische Schicht 20 aufgesetzt. Anschließend wird das Deckelsubstrat 12 mit der zweiten umlaufenden Schicht 22 so auf den umlaufenden Rahmen 50 aufgesetzt, dass die zweite umlaufende Schicht 22 auf den umlaufenden Rahmen 50 aufliegt. Anschließend wird der umlaufende Rahmen 50 mit den beiden metallischen Schichten 20, 22 unlösbar verbunden, was beispielsweise durch Verlöten der beiden metallischen Schichten 20, 22 mit dem umlaufenden Rahmen 50 erfolgen kann. In Fig. 1 ist das im Bereich zwischen den beiden umlaufenden Schichten 20, 22 aufgebrachte Lötzinn 60 erkennbar.

Dadurch wird ein geschlossenes Gehäusesystem gebildet, welches im Wesentlichen luft- und feuchtigkeitsdicht ist und in dessen Inneren sämtliche Elektronikkomponenten, insbesondere die Infrarot-Empfangs- bzw. -Sendeeinheit angeordnet sind.

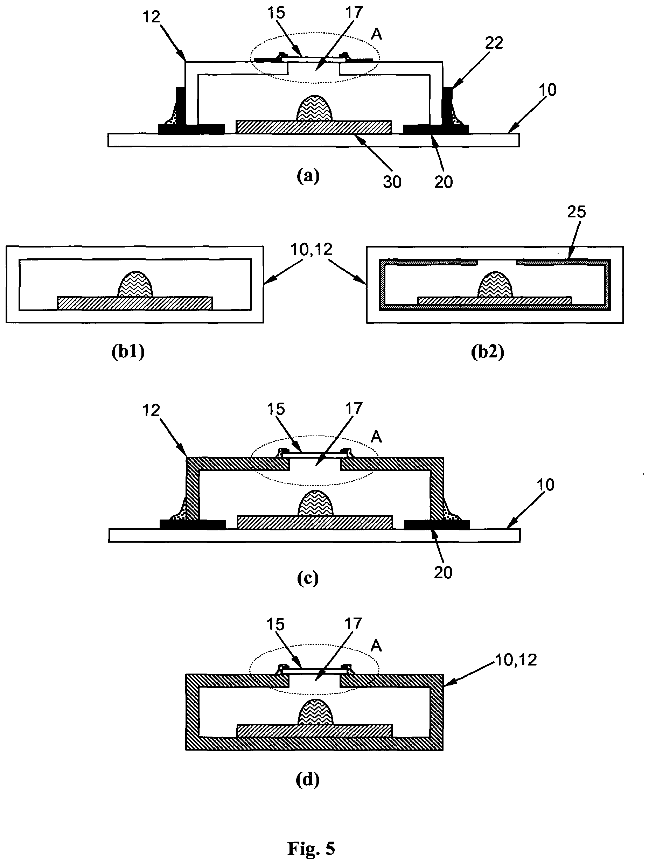

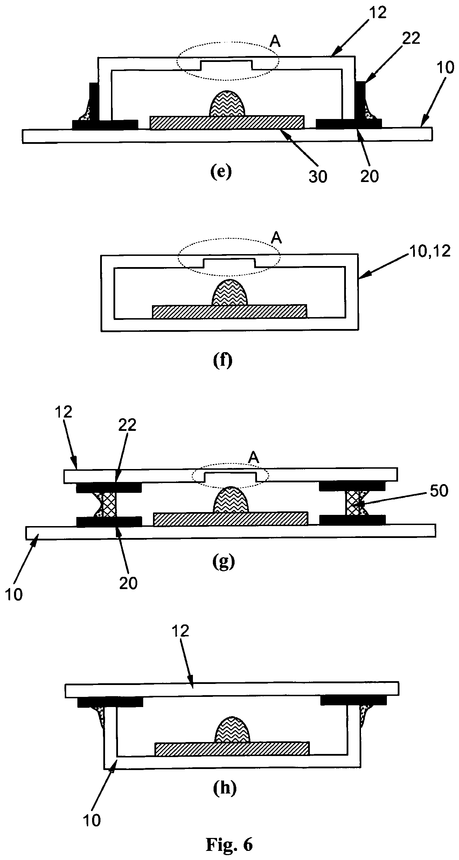

Das Deckelsubstrat 12 ist zumindest teilweise licht- bzw. infrarotlichtdurchlässig ausgestaltet, um eine Kommunikation mittels Infrarotstrahlung durch das Gehäuse hindurch mit einer externen Sende-/Empfangseinheit zu ermöglichen. Mögliche Ausgestaltungen eines licht- bzw. infrarotlichtdurchlässigen Bereiches einer Gehäusewandung werden mit Bezug auf Fig. 5 und Fig. 6 näher beschrieben.

**Fig. 2** zeigt eine zweite Variante eines erfindungsgemäßen Herstellungsverfahrens eines erfindungsgemäßen medizinischen Implantats, wobei die Herstellungsschritte S1 und S2 identisch zu dem in Fig. 1 gezeigten Herstellungsverfahren sind.

In einem dritten Schritt S3.2 der in Fig. 2 gezeigten zweiten Variante des Herstellungsverfahrens wird ein Deckelsubstrat 12, welches hier als Halbschale ausgebildet ist, an der Oberseite des Basissubstrats 12 angeordnet, sodass der Rand der Halbschale auf der umlaufenden metallischen Schicht 20 des Basissubstrats 10 aufliegt. An der äußeren Seitenwandung der Halbschale 12 ist eine umlaufende metallische Schicht 22 vorgesehen, welche bis an den Rand der Halbschale heranreicht und dadurch beim Aufsetzen der Halbschale auf das Basissubstrat 12 mit der umlaufenden metallischen Schicht 20 in Kontakt kommt.

In einem vierten Schritt S4.2 der zweiten Herstellungsvariante wird die erste rahmenförmige metallische Schicht 20 mit der zweiten rahmenförmigen metallischen Schicht 22 unlösbar miteinander verbunden, beispielsweise mittels eines Lötverfahrens. Das aufgebrachte Lötzinn 60 ist in Fig. 2 erkennbar. Das Deckelsubstrat 12 bzw. die Halbschale ist in einem Bereich licht- bzw. infrarotlichtdurchlässig ausgestaltet, wie mit Bezug auf Fig. 5 und Fig. 6 näher beschrieben wird.

Um das Deckelsubstrat licht- bzw. infrarotlichtdurchlässig auszugestalten, können für den lichtdurchlässigen bzw. infrarotlichtdurchlässigen Bereich des Deckelsubstrats unterschiedliche Materialien verwendet werden. Beispielsweise können unterschiedliche Keramiken in unterschiedlichen Dicken als Substratmaterial für das Deckelsubstrat verwendet werden. Beispiele solcher Keramiken sind etwa Aluminiumoxid Al₂O₃ in amorpher Form (Rubalit), in kristalliner Form (Saphir) oder in tcilkristallincr Form. Alternativ können auch Glas und Glaskeramiken verwendet werden. Ferner können auch Zirkoniumoxid ZrO₂ oder Hochtemperatur-Mehrlagenkeramiken (HTCC) mit einem variierenden Anteil an Aluminiumoxid Al₂O₃ zur Herstellung der licht- bzw. infrarotlichtdurchlässigen Bereiche verwendet werden.

Durch Anpassen der Dicke bzw. Stärke des Substratsmaterials im licht- bzw. infrarotlichtdurchlässigen Bereich kann die Infrarot-Transmission, d.h. die Transparenz für Infrarotstrahlung variiert werden.
Bei den mit Bezug auf Fig. 1 und Fig. 2 gezeigten Herstellungsverfahren kann auch ein hier nicht gezeigter Herstellungsschritt vorgesehen sein, in dem das Gehäuse mit einem elektrischen Leiter umwickelt wird, der eine Induktionsspule für eine induktive Energieübertragung bildet.

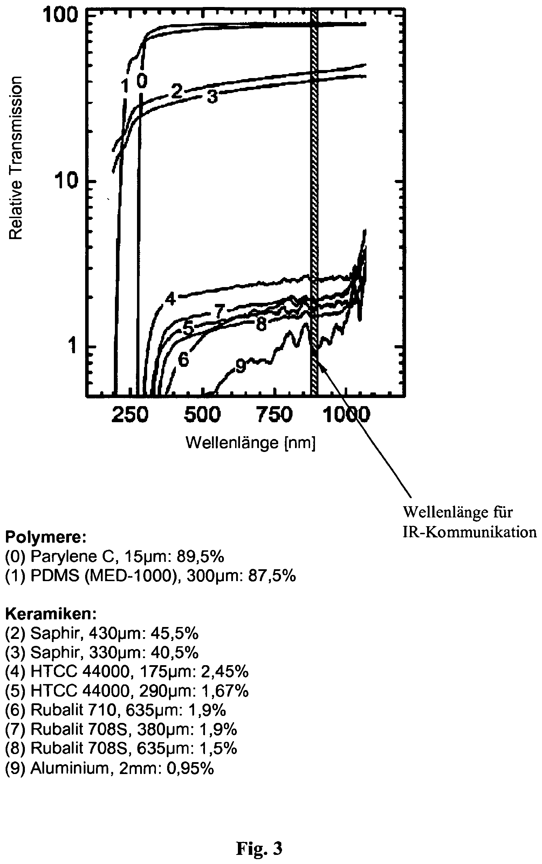

**Fig. 3** zeigt die relative Transmission von Infrarotlicht durch verschiedene Materialien in Abhängigkeit von der Wellenlänge des Infrarotlichtes und von dem verwendeten Material, wobei sich die Angaben der relativen Transmission bezüglich der einzelnen Materialien auf eine Wellenlänge der Infrarotstrahlung zwischen 850 nm und 900 nm beziehen.

In dem in Fig. 3 gezeigten Diagramm sind die relative Transmission für Rubalit, Saphir, und eine Hochtemperatur-Mehrlagenkeramik (HTCC) für jeweils unterschiedliche Stärken gezeigt. Ferner sind in dem Diagramm der Fig. 3 auch die relative Transmission für Aluminium bei einer Dicke von 2 mm, für Parylene C bei einer Dicke von 15 µm und für Polydimethylsiloxan, PDMS (MED-1000) bei einer Dicke von 300 µm aufgetragen.

Wie aus Fig. 3 ersichtlich, kann die Infrarot-Transparenz erhöht werden, indem das jeweilige Material dünner ausgestaltet wird. Ferner sind die Materialien umso transparenter für Infrarot-Strahlung je kristalliner das Material ist, wie ebenfalls aus Fig. 3 ersichtlich ist.

Zur luft- bzw. feuchtigkeitsdichten Verkapselung des Gehäuses eignen sich lötbare Metalle, etwa Kupfer oder Kupferfolie. Ebenfalls möglich sind hermetische Gehäuse aus Titan oder Edelstahl, wobei in diesen Gehäusen ein licht- bzw. infrarotlichtdurchlässiger Bereich vorgesehen sein muss (etwa Saphir, Rubin, Diamant oder ein ähnlicher fester licht- bzw. infrarotlichtdurchlässiger Kristall oder Edelstein, oder eine ähnlich licht- bzw. infrarotlichtdurchlässige Keramik), wie beispielsweise mit Bezug auf Fig. 5 gezeigt.

Als Lötzinn zum unlösbaren Verbinden des Deckels mit dem Boden des Gehäuses können alle gängigen Legierungen verwendet werden, wobei allerdings bleifreie Materialien zu bevorzugen sind.

Ferner kann es notwendig sein, das Gehäuse in einem medizinischen Elastomer zu vergießen bzw. das Gehäuse mit einem medizinischen Elastomer zu überziehen. Hierfür können gängige medizinische Silikone verwendet werden, beispielsweise Polydimethylsiloxan. Alternativ oder zusätzlich hierzu kann das Gehäuse mit einem Polymer, beispielsweise Parylene C überzogen bzw. verkapselt werden.

Die Infrarot-Übertragungseigenschaften werden sowohl durch das Polydimethylsiloxan als auch durch Parylene C nur unwesentlich beeinflusst, wie aus Fig. 3 ersichtlich ist.

**Fig. 4** zeigt ein erfindungsgemäßes System, welches ein medizinisches Implantat 1 und eine externe Sende-/Empfangseinheit 100 umfasst. Die Kommunikation zwischen dem Implantat 1 und der externen Sende-/Empfangseinheit 100 erfolgt auf Basis von Infrarotstrahlung. Das Deckelsubstrat 12 des Implantats 1 ist hierzu zumindest teilweise licht- bzw. infrarotlichtdurchlässig ausgestaltet, sodass eine Infrarot-Datenübertragung IR zwischen dem Implantat 1 und der externen Sende-/Empfangseinheit 100 möglich ist.

Das in Fig. 4 gezeigte Implantat 1 ist nach dem in Fig. 2 gezeigten Herstellungsverfahren hergestellt worden. Vor dem Verschließen des Gehäuses in den Schritten S3.2 bzw. S4.2 sind im Gehäuseinneren Magnete 70 angeordnet worden, welche als Permanentmagnete ausgestaltet sind. Die externe Sende-/Empfangseinheit 100, welche einen Infrarot-Sender und/oder InfrarotEmpfänger 40b aufweist, weist ebenfalls Magnete bzw. Permanentmagnete 71 auf, welche mit den Magneten 70 des Implantats 1 korrespondieren. Damit kann eine Ausrichtung der externen Sende-/Empfangseinheit 100 gegenüber dem implantierten Implantat 1 unterstützt werden. Gezeigt ist in Fig. 4 auch das zwischen den jeweiligen Magneten ausgebildete Magnetfeld MF.

Ferner kann vor dem Verschließen des Gehäuses in den Schritten S3.2 bzw. S4.2 im Gehäuseinneren eine Anzahl von Sensoren 80 angeordnet werden. Beispielsweise können ein Feuchtigkeitssensor, ein Temperatursensor und/oder ein Drucksensor vorgesehen werden. Die Sensoren sind mit der Infrarot-Sende-/Empfangseinrichtung 40 des Implantats 1 gekoppelt, sodass die Sensorwerte der Sensoren mittels IR-Kommunikation an die externe Sende-/Empfangseinheit 100 übertragen werden können.

Der Magnet 70 und die Sensoren 80 können auch in den Schritten S3.1 und S4.1 nach dem in Fig. 1 gezeigten Herstellungsverfahren im Inneren des Gehäuses angeordnet werden.

Mit einem Feuchtigkeitssensor, der vorzugsweise eine sehr geringe Drift aufweist, kann die Feuchtigkeit im Inneren des Implantatgehäuses in regelmäßigen Abständen überprüft werden. Überschreitet der Sensorwert des Feuchtigkeitssensors einen kritischen Wert, kann dies dem Anwender signalisiert werden, sodass dem Anwender aus Sicherheitsgründen ein Austausch des Implantats empfohlen werden kann. Dadurch wird es möglich, das Implantat solange wie möglich im Körper des Patienten zu belassen. Ein lediglich prophylaktischer Austausch eines Implantats kann so verhindert werden.

Zur Verlängerung der Zeit, in der die Feuchtigkeit innerhalb des Implantatgehäuses sehr gering ist und dadurch einen zuverlässigen Betrieb der im Gehäuseinneren angeordneten elektronischen Komponenten erlaubt, können Trocknungsmittel in das Gehäuse eingebracht werden, die in das Gehäuseinnere eindringende Wassermoleküle bis zu einer bestimmten Menge chemisch oder physikalisch binden, sodass sie nicht zur Erhöhung der Luftfeuchtigkeit im Gehäuseinneren beitragen können. Hierfür können sogenannte Getter-Materialien vorgesehen sein. Das Trocknungsmittel bzw. das Getter-Material kann auch an der Gehäuseinnenwandung aufgebracht werden, wobei die Infrarot-Transmission durch das Substratmaterial und das Trocknungsmittel bzw. das Getter-Material hindurch erfolgt.

Der Temperatursensor kann vorgesehen sein, um die Zuverlässigkeit der Elektronik innerhalb des Gehäuses weiter zu erhöhen. Der Temperatursensor kann beispielsweise verwendet werden, um eine thermische Überlastung des Implantats oder ein ungewolltes Aufwärmen des das Implantat umgebende Gewebe zu verhindern. Übersteigt die Temperatur einen vorbestimmten Wert, können durch das Implantat Gegenmaßnahmen eingeleitet werden. Beispielsweise kann die Funktion des Implantats auf eine Minimalfunktion reduziert werden. Die gemessenen Temperaturwerte können auch mittels IR-Kommunikation an die externe Sende-/Empfangseinheit 100 übermittelt werden. Bei häufigem Überschreiten bestimmter Temperaturwerte kann dem Patienten bzw. dem Anwender ein Austausch des Implantats nahegelegt werden.

**Fig. 5** zeigt fünf Varianten eines erfindungsgemäßen medizinischen Implantats für einen menschlichen oder tierischen Organismus.

Bei den Varianten in (a), (c) und (d) weist das Deckelsubstrat 12 eine Öffnung 17 auf, welche mit einer Abdeckung 15 verschlossen ist, wobei die Abdeckung 15 licht- bzw. infrarotlichtdurchlässig ausgestaltet ist. Als Materialien für die Abdeckung 15 können die vorstehend genannten Materialien verwendet werden. Das Deckelsubstrat 12 muss in diesen Ausführungsvarianten nicht licht- bzw. infrarotlichtdurchlässig ausgestaltet sein.

Die Abdeckung 15 wird auf das Deckelsubstrat 12 aufgelötet, wobei in Abhängigkeit von dem verwendeten Material für das Deckelsubstrat 12 auf dem Deckelsubstrat 12 und im randseitigen Bereich der Abdeckung 15 eine metallische Schicht aufgebracht ist, um ein Verlöten des Deckelsubstrats 12 mit der Abdeckung 15 zu ermöglichen. In der Variante (a) sind sowohl auf dem Deckelsubstrat 12 als auch auf der Abdeckung 15 entsprechende metallische Schichten aufgebracht. Bei der Variante (c) sind entsprechende metallische Schichten lediglich auf der Abdeckung 15 aufgebracht, weil nach dieser Variante das Deckelsubstrat metallisch ausgestaltet ist. Ebenso ist auch nach der Variante (d) lediglich auf der Abdeckung 15 eine entsprechende metallische Schicht vorgesehen.

Bei der Variante (d) ist sowohl das Deckelsubstrat 12 als auch das Basissubstrat 10 metallisch.

Gemäß Variante (b1) ist das Gehäuse einteilig ausgestaltet, wobei das gesamte Gehäuse 10, 12 ein licht- bzw. infrarotlichtdurchlässiges Material aufweist.

Die Variante (b2) unterscheidet sich von der Variante gemäß (b1) dadurch, dass an der Innenwandung des Gehäuses eine elektrische Abschirmung, etwa eine Metallfolie, aufgebracht ist, wobei die Abschirmung einen licht- bzw. infrarotlichtdurchlässigen Bereich aufweist. Der licht- bzw. infrarotlichtdurchlässige Bereich der Abschirmung 25 kann etwa durch eine Aussparung in der Metallfolie realisiert werden.

Die in der Variante (b2) gezeigte metallische Abschirmung kann bei sämtlichen der in Fig. 5 und Fig. 6 gezeigten Varianten vorgesehen werden.

**Fig. 6** zeigt vier weitere Varianten eines erfindungsgemäßen medizinischen Implantats.

Bei den Varianten (e), (f) und (g) ist das Deckelsubstrat 12 in dem Bereich, in dem das Deckelsubstrat licht- bzw. infrarotlichtdurchlässig ausgestaltet sein soll, dünner ausgestaltet als im übrigen Bereich des Deckelsubstrats. Die Dicke bzw.

Stärke des licht- bzw. infrarotlichtdurchlässigen Bereichs des Deckelsubstrats kann in Abhängigkeit von dem gewählten Substratmaterial gewählt werden, wie mit Bezug auf das Diagramm in Fig. 3 gezeigt.
Bei der Variante (h) sind die elektronischen Komponenten bzw. die Infrarot-Sende/Empfangseinrichtung 40 des Implantats nicht auf dem Basissubstrat 10 angeordnet, sondern auf dem Innenboden eines halbschalenförmig ausgestalteten Deckelsubstrats 12. In diesem Fall ist das Basissubstrat 10 an den entsprechenden Stellen licht- bzw. infrarotlichtdurchlässig auszugestalten wie mit Bezug auf die Varianten (a) bis (g) der Fig. 5 und 6 gezeigt.

### Bezugszeichen:

- 1: medizinisches Implantat
- 10: Substrat (Basissubstrat) als Teil des Gehäuses
- 12: Substrat (Deckelsubstrat) als Teil des Gehäuses
- 15: Abdeckung für eine Öffnung in der Gehäusewandung
- 17: Öffnung in der Gehäusewandung (Durchbruch)
- 20: erste rahmenförmige metallische Schicht auf dem Basissubstrat
- 22: zweite rahmenförmige metallische Schicht auf dem Deckelsubstrat
- 25: elektrische Abschirmung, z.B. Metallfolie
- 30: Platine (Elektronik-Platine)
- 40: Infrarot-Sendeeinrichtung und/oder Infrarot-Empfangseinrichtung des Implantats
- 40b: Infrarot-Sendeeinrichtung und/oder Infrarot-Empfangseinrichtung der externen Sende/Empfangseinheit
- 50: umlaufender Rahmen, vorzugsweise Metallrahmen als Teil des Gehäuses
- 60: unlösbare Verbindung zwischen der ersten und zweiten rahmenförmigen metallischen Schicht bzw. zwischen den rahmenförmigen metallischen Schichten und dem Metallrahmen, vorzugsweise Lötzinn
- 70: Magnet bzw. Permanentmagnet in dem Implantat
- 71: Magnet bzw. Permanentmagnet an der externen Infrarot-Sende/Empfangseinheit
- 80: Sensor, z.B. Feuchtigkeitssensor und/oder Temperatursensor und/oder Drucksensor
- 100: externe IR-Sende/Empfangseinheit
- A: Ausschnitt der Gehäusewandung, in dem sich der lichtdurchlässige Bereich befindet
- HTCC: Hochtemperatur-Mehrlagenkeramik (engl. High Temperature Cofired Ceramics)
- IR: Infrarot Datenübertragung
- MF: Magnetfeld
- O: Oberseite des Basissubstrats
- S1: erster Schritt der Herstellungsverfahren
- S2: zweiter Schritt der Herstellungsverfahren
- S3.1: dritter Schritt des Herstellungsverfahrens gemäß einer ersten Variante
- S3.2: dritter Schritt des Herstellungsverfahrens gemäß einer zweiten Variante
- S4.1: vierter Schritt des Herstellungsverfahrens gemäß einer ersten Variante
- S4.2: vierter Schritt des Herstellungsverfahrens gemäß einer zweiten Variante

## Patentansprüche

1. Medizinisches Implantat für einen menschlichen oder tierischen Organismus, umfassend ein Gehäuse mit einer Gehäusewandung, welche zumindest einen lichtdurchlässig, insbesondere infrarotlichtdurchlässig, ausgestalteten Bereich (A) aufweist, und eine im Gehäuseinneren angeordnete Infrarot-Sendeeinrichtung und/oder Infrarot-Empfangseinrichtung (40) für eine Kommunikation zwischen Implantat und externer Sende-/Empfangseinrichtung,
**dadurch gekennzeichnet, dass** der lichtdurchlässig ausgestaltete
Bereich
- Aluminiumoxid, Al₂0₃, in amorpher, und/oder teilkristalliner Form oder Zirkoniumoxid, Zr0₂, in amorpher, und/oder teilkristalliner Form, oder
- Hochtemperatur-Mehrlagenkeramik mit einem Aluminiumoxid-Anteil
aufweist und
wobei der lichtdurchlässig ausgestaltete Bereich (A) des Gehäuses eine geringere Stärke aufweist als der restliche Bereich des Gehäuses.

2. Medizinisches Implantat nach Anspruch 1, wobei der lichtdurchlässig ausgestaltete Bereich durch eine Abdeckung (15) gebildet wird, die eine Öffnung (17) in der Gehäusewandung verschließt.

3. Medizinisches Implantat nach einem der vorherigen Ansprüche, wobei in dem Gehäuseinneren zumindest ein Sensor (80), insbesondere ein Feuchtigkeitssensor und/oder ein Temperatursensor angeordnet ist, der operativ mit der Infrarol-Sendeeinrichtung und/oder Infrarot-Empfangseinrichtung (40) gekoppelt ist.

4. Medizinisches Implantat nach einem der vorherigen Ansprüche, wobei im Gehäuseinneren zumindest ein Magnet (70), insbesondere Permanentmagnet angeordnet ist, der mit einem außerhalb des Gehäuses anordenbaren Magneten (70) einer externen Sende/Empfangseinheit (100) zusammenwirken kann.

5. Medizinisches Implantat nach einem der vorherigen Ansprüche, wobei das Implantat eine Induktionsspule aufweist, die induktiv mit einer außerhalb des Körpers anordenbaren induktiven Sendeeinheit koppelbar ist, um Energie von der induktiven Sendeeinheit an das Implantat zu übertragen.

6. Medizinisches Implantat nach Anspruch 5, wobei die Induktionsspule einen elektrischen Leiter umfasst, der um das Gehäuse des Implantats gewickelt ist.

7. Medizinisches Implantat nach einem der vorherigen Ansprüche, wobei im Gehäuseinneren ein Trocknungsmittel vorgesehen ist.

8. Verfahren zur Herstellung eines medizinischen Implantats (1), nach einem der Ansprüche 1 bis 7, wobei
- auf einer Oberseite (0) eines Basissubstrats (10) eine im Wesentlichen erste rahmenförmige metallische Schicht (20) aufgebracht wird (S 1),
- auf der Oberseite des Basissubstrats (10) und innerhalb der ersten rahmenförmigen metallischen Schicht (20) eine Infrarot-Sendeeinrichtung und/oder Infrarot-Empfangseinrichtung (40) angeordnet wird (S2),
- auf der Oberseite des Basissubstrats (10) ein Deckelsubstrat (12) angeordnet wird (S3), welches den Bereich innerhalb der ersten rahmenförmigen metallischen Schicht (20) abdeckt und welches zumindest einen lichtdurchlässig, insbesondere infrarotlichtdurchlässig ausgestalteten Bereich aufweist, wobei an dem Deckelsubstrat (12) eine zweite, mit der ersten rahmenförmige metallischen Schicht (20) korrespondierende, rahmenförmige metallische Schicht (22) vorgesehen wird, und
- die erste rahmenförmige metallische Schicht (20) derart mit der zweiten rahmenförmigen metallischen Schicht (22) unlösbar verbunden wird, dass das Basissubstrat (10), das Deckelsubstrat (12) und die rahmenförmigen metallischen Schichten (20, 22) zusammen ein im Wesentlichen hermetisch geschlossenes Gehäuse des Implantats bilden.

9. Verfahren nach Anspruch 8, wobei zwischen der ersten rahmenförmigen metallischen Schicht (20) und der zweiten rahmenförmigen metallischen Schicht (22) ein umlaufender Rahmen (50), vorzugsweise Metallrahmen angeordnet wird und wobei der Rahmen mit den beiden metallischen Schichten (20, 22) unlösbar verbunden wird.

10. Verfahren nach Anspruch 8 oder 9, wobei der lichtdurchlässig ausgestaltete Bereich durch eine Abdeckung (15) gebildet wird, mit der eine Öffnung (17) in dem Deckelsubstrat (12) verschlossen wird.

11. Verfahren nach Anspruch 8 oder 9, wobei das Deckelsubstrat (12) so ausgestaltet wird, dass der lichtdurchlässige Bereich eine geringe Stärke aufweist als der restliche Bereich des Deckelsubstrats (12).

12. Verfahren nach einem der Ansprüche 8 bis 11, wobei für den lichtdurchlässigen Bereich
- Aluminiumoxid, Al₂O₃ in amorpher, und/oder teilkristalliner Form oder Zirkoniumoxid, ZrO₂. in amorpher und/oder teilkristalliner Form, oder
- Hochtemperatur-Mehrlagenkeramik, HTCC, mit einem Aluminiumoxid-Anteil,
verwendet wird.

13. system, umfassend ein medizinisches Implantat (1) für einen menschlichen oder tierischen Organismus nach einem der Ansprüche 1 bis 7 und eine Sende/Empfangseinheit (100), die eine Infrarot-Sendeeinrichtung und/oder Infrarot-Empfangseinrichtung (40b) aufweist, wobei eine Infrarot-Sendeeinrichtung und/oder Infrarot-Empfangseinrichtung (40) des Implantats durch einen lichtdurchlässig, insbesondere infrarotlichtdurchlässig ausgestalteten Bereich des Gehäuses des Implantats hindurch operativ mit der Infrarot-Sendeeinrichtung und/oder Infrarot-Empfangseinrichtung (40b) der Sende/Empfangseinheit (100) koppelbar ist.

## Claims

1. Medical implant for a human or animal organism, comprising a housing having a housing wall that has at least one region (A) configured to be permeable to light, in particular permeable to infrared light, and an infrared transmitting device and/or infrared receiving device (40) arranged in the interior of the housing and permitting communication between the implant and an external transmitting/receiving device, **characterised in that** said region configured to be permeable to light has
- aluminium oxide, Al₂O₃, in an amorphous and/or semi-crystalline form, or zirconium oxide, ZrO₂ in an amorphous and/or semi-crystalline form, or
- high-temperature multilayer ceramic with some aluminium oxide content, and
with the region (A) of the housing that is configured to be permeable to light having a smaller thickness than the remaining region of the housing.

2. The medical implant as claimed in claim 1, wherein the region configured to be permeable to light is formed by a lid (15) that seals an opening (17) provided in the housing wall.

3. The medical implant as claimed in any of the preceding claims, wherein at least one sensor (80), in particular a humidity sensor and/or a temperature sensor, is arranged in the interior of the housing and is operatively coupled to the infrared transmitting device and/or infrared receiving device (40).

4. The medical implant as claimed in any of the preceding claims, wherein at least one magnet (70), in particular a permanent magnet, is arranged in the interior of the housing and may cooperate with a magnet (70) of an external transmitting/receiving unit (100) which is located outside of the housing.

5. The medical implant as claimed in any of the preceding claims, wherein the implant has an induction coil that may be inductively coupled to an inductive transmitting unit that may be arranged outside the body in order to transmit energy from the inductive transmitting unit to the implant.

6. The medical implant as claimed in claim 5, wherein the induction coil comprises an electrical conductor that is wound around the housing of the implant.

7. The medical implant as claimed in any of the preceding claims, wherein a desiccant is provided in the interior of the housing.

8. A method for producing a medical implant (1) as claimed in any one of claims 1 to 7, wherein
- on an upper surface (0) of a base substrate (10), a first essentially frame-shaped metallic layer (20) is applied (S1),
- on said upper surface of said base substrate (10) and within said first frame-shaped metallic layer (20), an infrared emitting device and/or infrared receiving device (40) is arranged (S2),
- on said upper surface of said base substrate (10), a cover substrate (12) is arranged (S3) which covers the region within said first frame-shaped metallic layer (20) and which has a least one region configured to be permeable to light, in particular permeable to infrared light, a second frame-shaped metallic layer (22) coinciding with said first frame-shaped metallic layer (20) being provided on the cover substrate (12), and
- said first frame-shaped metallic layer (20) is undetachably connected to said second frame-shaped metallic layer (22) in such a manner that the base substrate (10), the cover substrate (12) and the frame-shaped metallic layers (20, 22) cooperate to form an essentially hermetically sealed housing of the implant.

9. The method as claimed in claim 8, wherein a circumferential frame (50), preferably a metallic frame, is arranged between said first frame-shaped metallic layer (20) and said second frame-shaped metallic layer (22), said frame being undetachably connected to both metallic layers (20, 22).

10. The method as claimed in claim 8 or 9, wherein the region configured to be permeable to light is formed by a lid (15) by means of which an opening (17) provided in the cover substrate (12) is sealed.

11. The method as claimed in claim 8 or 9, wherein the cover substrate (12) is configured in such a manner that the translucent region has a smaller thickness than the remaining region of the cover substrate (12).

12. The method as claimed in any one of claims 8 to 11, wherein for the translucent region
- aluminium oxide, Al₂O₃, in an amorphous and/or semi-crystalline form, or zirconium oxide, ZrO₂, in an amorphous and/or semi-crystalline form, or
- high-temperature multilayer ceramic with some aluminium oxide content
is used.

13. A system comprising a medical implant (1) for a human or animal organism as claimed in any one of claims 1 to 7 and a transmitting/receiving unit (100) which has an infrared transmitting device and/or infrared receiving device (40b), wherein an infrared transmitting device and/or infrared receiving device (40) of the implant may be operatively coupled to the infrared transmitting device and/or infrared receiving device (40b) of the transmitting/receiving unit (100) through a region of the implant housing configured to be permeable to light, in particular permeable to infrared light.

## Revendications

1. Implant médical pour un organisme humain ou animal, comprenant un boîtier avec une paroi de boîtier laquelle présente au moins une zone (A) conçue pour laisser passer la lumière, en particulier la lumière infrarouge, et un dispositif émetteur infrarouge et/ou dispositif récepteur infrarouge (40) disposé à l'intérieur dudit boîtier pour assurer une communication entre l'implant et un dispositif émetteur/récepteur externe, **caractérisé en ce que** la zone conçue transparente présente
- de l'oxyde d'aluminium, Al₂O₃, sous forme amorphe et/ou semi-cristalline ou de l'oxyde de zirconium, ZrO₂, sous forme amorphe et/ou semi-cristalline, ou
- de la céramique multicouche haute température avec une teneur donnée en oxyde d'aluminium et
la zone (A) du boîtier conçue pour laisser passer la lumière présentant une épaisseur plus faible que la zone restante du boîtier.

2. Implant médical selon la revendication 1, dans lequel la zone conçue pour laisser passer la lumière est formée par un élément de recouvrement (15) qui ferme une ouverture (17) ménagée dans la paroi de boîtier.

3. Implant médical selon l'une quelconque des revendications précédentes, dans lequel au moins un capteur (80), en particulier un capteur d'humidité et/ou un capteur de température, qui est couplé de manière opérationnelle au dispositif émetteur infrarouge et/ou dispositif récepteur infrarouge (40) est disposé à l'intérieur du boîtier.

4. Implant médical selon l'une quelconque des revendications précédentes, dans lequel au moins un aimant (70), en particulier un aimant permanent, est disposé à l'intérieur du boîtier et peut coopérer avec un aimant (70) d'une unité émettrice/réceptrice externe (100) lequel peut être disposé à l'extérieur du boîtier.

5. Implant médical selon l'une quelconque des revendications précédentes, dans lequel l'implant présente une bobine d'induction qui peut être couplée de manière inductive à une unité émettrice inductive pouvant être disposée à l'extérieur du corps afin de transmettre de l'énergie, et ce de l'unité émettrice inductive à l'implant.

6. Implant médical selon la revendication 5, dans lequel la bobine d'induction comprend un conducteur électrique qui est enroulé autour du boîtier.

7. Implant médical selon l'une quelconque des revendications précédentes, dans lequel un agent desséchant est prévu à l'intérieur du boîtier.

8. Procédé destiné à la fabrication d'un implant médical (1) selon l'une quelconque des revendications 1 à 7, lors duquel
- une première couche métallique (20) essentiellement en forme de cadre est appliquée sur une face supérieure (0) d'un substrat de base (10) (S1),
- un dispositif émetteur infrarouge et/ou dispositif récepteur infrarouge (40) est disposé sur la face supérieure du substrat de base (10), et ce à l'intérieur de la première couche métallique (20) en forme de cadre (S2),
- un substrat couvercle (12) qui recouvre la zone située à l'intérieur de la première couche métallique (20) en forme de cadre et qui présente au moins une zone conçue pour laisser passer la lumière, en particulier la lumière infrarouge, est disposé sur la face supérieure du substrat de base (10) (S3), une deuxième couche métallique (22) en forme de cadre, laquelle coïncide avec la première couche métallique (20) en forme de cadre, étant prévue sur le substrat couvercle (12), et
- la première couche métallique (20) en forme de cadre est unie de manière indissociable à la deuxième couche métallique (22) en forme de cadre de manière telle que le substrat de base (10), le substrat couvercle (12) et les couches métalliques (20, 22) en forme de cadre forment ensemble un boîtier d'implant fermé essentiellement de manière hermétique.

9. Procédé selon la revendication 8, lors duquel un cadre circonférentiel (50), de préférence un cadre en métal, est disposé entre la première couche métallique (20) en forme de cadre et la deuxième couche métallique (22) en forme de cadre et lors duquel le cadre est relié de manière indissociable aux deux couches métalliques (20, 22).

10. Procédé selon la revendication 8 ou 9, lors duquel la zone conçue pour laisser passer la lumière est formée par un élément de recouvrement (15) qui ferme une ouverture (17) ménagée dans le substrat couvercle (12).

11. Procédé selon la revendication 8 ou 9, lors duquel le substrat couvercle (12) est conçu de telle sorte que la zone translucide présente une épaisseur plus faible que la zone restante du substrat couvercle (12).

12. Procédé selon l'une quelconque des revendications 8 à 11, lors duquel les éléments utilisés pour la fabrication de la zone translucide sont :
- l'oxyde d'aluminium, Al₂O₃, sous forme amorphe et/ou semi-cristalline ou l'oxyde de zirconium, ZrO₂, sous forme amorphe et/ou semi-cristalline, ou
- la céramique multicouche haute température, HTCC, avec une teneur donnée en oxyde d'aluminium.

13. Système comprenant un implant médical (1) pour un organisme humain ou animal selon l'une quelconque des revendications 1 à 7 et une unité émettrice/réceptrice (100) qui présente un dispositif émetteur infrarouge et/ou dispositif récepteur infrarouge (40b), système dans lequel un dispositif émetteur infrarouge et/ou dispositif récepteur infrarouge (40) de l'implant peut être couplé de manière opérationnelle au dispositif émetteur infrarouge et/ou dispositif récepteur infrarouge (40b) de l'unité émettrice/réceptrice (100), et ce à travers une zone du boîtier de l'implant conçue pour laisser passer la lumière, en particulier la lumière infrarouge.
